# EUROPEAN PATENT SPECIFICATION

(11) **EP 1 973 513 B1**
(45) Date of publication and mention of the grant of the patent: **10.11.2010**
(21) Application number: 06848115.9
(22) Date of filing: 22.12.2006
(51) Int. Cl.: A61K 6/097, A61L 27/36, A61L 27/50, A61L 27/46, A61L 27/58

(54) **LIGHT-CURABLE BONE GROWTH MATERIAL FOR TREATING DENTAL BONE DEFECTS**
LICHTHÄRTBARES KNOCHENWACHSTUMSMATERIAL ZUR BEHANDLUNG VON ZAHNKNOCHENDEFEKTEN
MATÉRIAU DE CROISSANCE OSSEUSE PHOTOPOLYMÉRISABLE POUR LE TRAITEMENT DES DÉFAUTS OSSEUX DENTAIRES

(30) Priority: 28.12.2005 US 754453 P
(43) Date of publication of application: 01.10.2008
(73) Proprietor: DENTSPLY International Inc., York, PA 17405-0872 (US)
(72) Inventor: WALLINE, Katherine, Suzanne, Boulder, CO 80305 (US); ATKINSON, Brent, Lee, Frederick, MD 21704 (US)
(74) Representative: Hartz, Nikolai
(86) International application number: PCT/US2006/049198
(87) International publication number: WO 2007/079053

(56) References cited:
- WO-A-2004/011053
- WO-A-2004/075862
- US-A- 5 837 752

## Description

### BACKGROUND OF THE INVENTION

### Field of the Invention

The present invention relates generally to compositions for promoting growth of new bone. The compositions contain a mixture of bone particulate and polymeric carrier having polymerizable groups. The carrier can be light-cured to form a cross-linked, biodegradable hydrogel. The composition is particularly suitable for dental and orthopedic applications. Methods of applying the bone growth-inducing material to defective bone tissue are also provided.

### Brief Description of the Related Art

In general, it is known that biodegradable polymeric networks can be used as implant materials and carriers for biologically active materials. For instance, a polymerizable material can be applied to defective bone tissue or other area in the body of the subject (human or other species) where an implant is needed. Then, the polymerizable material can be polymerized to form a hardened shaped implant. Polymeric implants such as, for example, rods, pins, screws, and plates can be made according to such methods. The implants can be used in dental and orthopedic applications. To cure or harden the polymeric implants, the material is irradiated with light and/or heat, or a self-curing mechanism such as a redox reaction is initiated. The hardened polymeric implants generally have good mechanical strength and are compatible with surrounding tissue. One advantage of such *in vivo* polymerizable materials is that the material is flexible and can be molded and fabricated into complex shapes during implantation. The polymerizable material can be applied to the implant site and cured *in vivo* to produce a hardened implant having specific geometric dimensions. Alternatively, the materials can be injection-molded or otherwise shaped and polymerized ex *vivo* and then implanted. The hardened polymer degrades at a controlled rate depending upon its composition. Over time, it is expected that most of the polymeric implant will be replaced by new bone.

Hydrogel materials, in contrast to harder polymerizable materials, provide the advantage of good malleability prior to curing as well as better resorption and biocompatibility. One weakness of hydrogels for these applications is their lower mechanical strength post-cure.

In other instances, the biodegradable polymeric networks are used as carriers for biologically active materials such as, for example, hormones, enzymes, antibiotics, antiinflammatory agents, and growth factors including osteotherapeutic materials. The polymeric network is cross-linked, by photopolymerization or other mechanism, to create a stable hydrogel that will retain water. Over time, the cross-linked hydrogel will degrade under normal physiological conditions and will be resorbed and metabolized by the body.

For example, Anseth et al., U.S. Patent 5,902,599 discloses biodegradable cross-linked networks that are formed by cross-linking functionalized anhydride monomers or oligomers. The polymeric networks can be used in dental and orthopedic applications. According to the '599 Patent, useful functionalized monomers or oligomers include mixed anhydrides of a diacid and a carboxylic acid molecule that includes a cross-linkable group such as an unsaturated moiety. Exemplary anhydride monomers or oligomers include mixed anhydrides of diacids, such as sebacic acid or 1,6-bis(p-carboxyphenoxy)-hexane (MCPH), and a carboxylic acid including an unsaturated moiety such as methacrylic acid. The functionalized anhydride monomers or oligomers are formed, for example, by reacting the diacid with an activated form of the acid, such as an anhydride thereof, to form a mixed anhydride. The cross-linked polymer network can be formed *in vivo* by irradiating the anhydride monomers or oligomers with ultraviolet or visible light.

Hubbell et al., U.S. Patents 5,410,016 and 5,626,863 disclose biocompatible, biodegradable hydrogels that can be polymerized *in vivo*. The hydrogels are formed from macromers, which include at least one water-soluble region, at least one region which is biodegradable, usually by hydrolysis, and at least two free-radical polymerization regions. In a particularly preferred embodiment, the macromer includes a core made of hydrophilic poly(ethylene glycol) oligomers, an extension on each end of the core, the extension made of poly(lactic acid) oligomers, and end caps made of acrylate moieties which are capable of cross-linking and polymerization. Photopolymerization using visible or ultraviolet radiation can be used to polymerize the macromer. The hydrogel materials can be used to prevent adhesions from forming after surgical procedures, for controlled drug delivery, temporary protection or separation of tissue surfaces, adhering of sealing tissues together, and preventing the attachment of cells to tissue surfaces.

Chudzik et al., U.S. Patent 6,007,833 discloses a cross-linkable macromer system comprising two or more polymer-pendant polymerizable groups and one or more polymer-pendant initiator groups. Ultraviolet or visible light radiation can be used to polymerize the macromer system. In a preferred embodiment, the polymerizable groups and initiator groups are pendant on the same polymeric backbone. In an alternative embodiment, the polymerizable groups and initiator groups are pendant on different polymeric backbones. The macromer system can be used in such applications as controlled drug release, preparation of tissue adhesives and sealants, immobilization of cells, and preparation of three-dimensional bodies for implants.

Lin et al., Published United States Patent Application US 2004/0091462 discloses compositions for delivering osteotherapeutic materials to viable bone and/or other skeletal tissues to repair defects. Such osteotherapeutic materials include osteoinductive and/ or osteoconductive materials such as, for example, demineralized bone matrix and cortical-cancellous bone chips. Macromers, containing at least one water-soluble block, at least one biodegradable block, and at least one polymerizable group are used as carriers for the osteotherapeutic materials. Di-block macromers may include a water-soluble block linked to a biodegradable block, with one or both ends capped with a polymerizable group. Tri-block macromers may include a central water-soluble block and outside biodegradable blocks, with one or both ends capped with a polymerizable group. Polymerization mechanisms include photopolymerization, redox reactions, and cationic polymerization. In one embodiment, the carrier is a macromer of poly(ethylene glycol), trimethylene carbonate (TMC), and poly(lactic acid). The composition may be polymerized into a pre-selected shape and used as an implant for promoting bone growth.

Bone grafting materials are implanted into the body using various techniques to help the body make new bone according to various mechanisms. Many compositions containing bone grafting material and biodegradable, polymeric carriers are known in the art. Over time, the biodegradable, polymeric carrier is resorbed by the body and replaced by new bone. It is particularly important that an advanced bone grafting material be used in dentistry and oral surgery. Such bone grafting materials may be used in the treatment of intrabony periodontal osseous defects due to moderate or severe periodontitis, augmentation of bony defects of the alveolar ridge, filling tooth extraction sites, or sinus elevation grafting. Ideally, the bone grafting material should include inorganic and organic components that mimic autogenous bone. Compositions containing bone grafting materials that demonstrate high compressive strength are also desirable. In addition, the compositions should have good handling properties and be capable of being cured in *vivo* using polymerization mechanisms to form a stable hydrogel. With such a composition, the clinician would be able to mold the composition to a particularly desired shape and cure the structure *in vivo*. The present invention provides such bone grafting compositions having these desirable properties as well as other beneficial features and advantages.

### SUMMARY OF THE INVENTION

The invention provides improved compositions for promoting growth of new bone material. The composition comprises porous, resorbable particulate derived from anorganic bone material; a resorbable, biocompatible carrier gel material comprising photopolymerizable polysaccharide having polymerizable groups, said material forming a stable hydrogel matrix upon polymerization, said particulate being dispersed within said hydrogel; and a polymerization system that is activated by light to polymerize the carrier material. Preferably, the particulate is bovine derived and has an average particle size in the range of 250 µm to 1000 µm. More preferably, the particle size is in the range of 250 to 420 µm. The particulate is generally present in the composition in an amount in the range of 30% to 75% by weight based on weight of the composition. The particulate has a relatively high density. For example, the composition can contain 40% to 60% particulate having a bulk density of about 1.1 to about 1.3 g/cc. Preferably, the photopolymerizable polysaccharide is, for example, methacrylated sodium hyaluronate (MHy) or methacrylated hydroxyethylcellulose (MHEC). Mixtures of MHy and MHEC also can be used. The carrier gel normally contains 2% to 10% methacrylated polysaccharide by weight. The polymerization system comprises a photopolymerization initiator. Polymerization accelerators such as tertiary amines also can be added. In a preferred embodiment, the polymerization system comprises a blend of Eosin Y, triethanolamine, and N-vinyl caprolactam.

The composition is preferably in the form of putty having good viscosity and handling properties. A clinician can mold and shape the composition to a desired structure at the bone repair site. The composition has good dimensional stability so it does not expand or shrink substantially from the site. Then, the composition can be cured in situ using a photopolymerization process. The polymerization system of the composition can be activated by blue, visible light having a wavelength in the range of 400 to 600 nm. Standard dental curing lamps can be used to generate this irradiation. Because the particulate and hydrogel carrier are resorbable, the composition is eventually replaced by new bone.

### BRIEF DESCRIPTION OF THE FIGURES

FIG. 1 is a bar graph showing the compressive modulus properties of materials made with DFDBA versus materials made with OG/N-300 particulate at various concentration levels;

FIG. 2 is a bar graph showing the compressive modulus properties of materials made with DFDBA versus materials made with OG/N-300 particulate at various cure times;

FIG. 3 is a bar graph showing the compressive modulus properties of materials made with OG/N-300 particulate at various concentration levels and cure times;

FIG. 4 is a bar graph showing the compressive modulus properties of materials made with GMHy carrier and OG/N-300 particulate at various concentration levels; and

FIG. 5 shows the histology of bone formation after implantation of MHEC/ABM/P-15 bone growth material.

### DETAILED DESCRIPTION OF THE PREFERRED EMBODIMENTS

The present invention relates to an improved composition for promoting new bone growth. The composition according to claim 1 contains particulate bone grafting material and a light-curable biodegradable, polymeric carrier.

Various bone grafting materials are known generally in the art and such materials are often referred to as osteotherapeutic materials. Examples of bone grafting materials include, but are not limited to, demineralized bone matrix ("DBM"), demineralized freeze dried bone allograft ("DFDBA"), cortical-cancellous bone chips ("CCC"), bone morphogenic proteins ("BMP"), growth factors and hormones such as, for example, platelet-derived growth factor ("PDGF"); fibroblast growth factors ("FGFs"); cell suspensions, synthetic peptides, autogenous bone, hydroxyapatite ("HA"); tricalcium phosphate ("TCP"); other calcium phosphates; calcium carbonate; calcium sulfate; collagen; and the like.

Of the numerous bone grafting materials known in the art, the inventors have found that a porous, resorbable particulate derived from anorganic bone matrix ("ABM") is particularly effective in making the composition of this invention. The anorganic bone matrix particulate is preferably bovine-derived and has a particle size in the range of 250 to 1000 µm and more preferably in the range of 250 to 420 µm. The anorganic bone matrix particulate comprises hyroxyapatite mineral. Such a particulate material is commercially available as OsteoGraf//N-300™ from Dentsply Friadent Ceramed (Lakewood, CO). The OsteoGraf/N-300™ particulate may be referred to as ABM or OG/N-300 in the following examples.

In a preferred embodiment, a synthetic polypeptide sequence known as "P-15" is bound to the anorganic bone matrix particulate. Such a particulate material containing the P-15 polypeptide is commercially available as PepGen P-15™ from Dentsply Friadent Ceramed (Lakewood, CO). In the PepGen P-15™ product, the P-15 polypeptide material is bound irreversibly to the hydroxyapatite particulate - this allows the P-15 polypeptide material to remain active. The PepGen P-15™ material may be referred to as ABM/P-15 in the following examples. Methods used to synthesize the P-15 polypeptide materials and bind them to the hydroxyapatite particulate are described in Bhatnager, U.S. Patents 5,354,736 and 5,635,482, the disclosures of which are hereby incorporated by reference. The OsteoGraf/N-300™ and PepGen P-15™ bone growth materials offer several advantages over conventional bone grafting materials.

For example, both bone growth materials are compatible with the polymeric carrier and provide a stable platform for new bone growth. The PepGen P-15™ material is particularly effective because of its similar properties to natural bone. Autogenous bone has two basic components, organic and inorganic. The inorganic component of autogenous bone is primarily a microporous calcium phosphate mineral (hydroxyapatite). The organic component of autogenous bone is primarily collagen, particularly Type-I collagen, which constitutes three strands of 1300 amino acids that are helically intertwined. Within this amino acid chain, it has been found that a specific binding sequence of 15 amino acids plays a significant role in bone regeneration. PepGen P-15™ is a synthetic analog of the 15 amino acid sequence (P-15), which is irreversibly attached to the hydroxyapatite minerals. Thus, PepGen P-15™ mimics the inorganic and organic components of autogenous bone. The inorganic component has the same mineral composition as human bone providing a natural scaffold for bone regeneration. The organic component mimics collagen and enhances the binding of bone regenerative cells while promoting proliferation and differentiation of the cells as needed for new bone formation.

It also has been found that the photopolymerizable hydrogel/particulate materials, which contain the anorganic bone matrix particulate (OsteoGraflN-300™ and PepGen P-15™) have greater compressive modulus versus compositions containing other bone grafting materials, particularly DFDBA, as described further below. The compressive strength of the composition indicates the composition's resistance to being deformed when a load is applied to the material. Dental materials having high compressive strengths are desirable, because they are able to withstand high mastication forces or forces caused by soft tissue suturing over the wound site. One possible explanation for the higher compressive strength is that the anorganic bone matrix particles found in these compositions have a relatively high density.

The particulate derived from the anorganic bone matrix material is normally present in the composition of this invention in an amount in the range of about 30% to about 75%. In one preferred version, the particulate has a bulk density of about 1.1 to about 1.3 g/cc and the composition comprises about 40 to about 60 wt.% particulate. When particulate having these bulk density values is used at these concentrations, the resulting composition has a putty-like consistency and is moldable and easy to handle. The putty composition can be placed in a dental bony defect and molded to a desired shape while remaining substantially fixed in place. It has good dimensional stability as it neither significantly expands into neighboring healthy bone tissue nor shrinks at the site of bone repair. By contrast, particulate used in other bone growth materials, for example, DFDBA, may have a bulk density that is too low. The resulting composition may be too thin so that it cannot be easily molded or the cured composition may be too brittle and crumble in the bone repair site.

The high density particles used in this invention also may provide the putty composition with a higher refractive index. Light irradiation, which is directed onto the composition, is not blocked by the particles; rather, it is refracted. As a result, curing light irradiation can penetrate more deeply into the composition so that it is more fully and uniformly cured.

The carrier gel for the particulate bone grafting material is a biocompatible and biodegradable polysaccharide containing photopolymerizable groups. Preferably by photopolymerization using ultraviolet or visible light radiation. Examples of polymerizable groups include acrylates, methacrylates, acrylamides, methacrylamides, and styrene. The polymeric carrier is polymerized and cross-linked to form a stable hydrogel matrix in accordance with this invention. The particles are uniformly dispersed in and held in place by the hydrogel matrix so that they do not migrate away from the bone repair site. In addition, the hydrogel matrix provides adequate spacing between the particles. Over packing of the particles is prevented and this allows new cell and vascular infiltration at the bone repair site to occur. The hydrogel matrix provides a temporary support for new bone growth. The particulate and carrier materials are resorbable so, over time, they will be turned over into natural bone. Eventually, these materials are completely integrated by new bone tissue. Because the particulate and carrier materials of this invention have good biodegradation rates, new bone formation is enhanced. If the biodegradation rate is too fast, cellular and vascular infiltration cannot develop at the site and this prevents bone formation. On the other hand, if the biodegradation rate is too slow, this also will interfere with cellular migration and vascular penetration.

As mentioned above, there are many known biodegradable carriers containing polymerizable groups that are used for delivering bone grafting materials. Of the many possible biodegradable carriers that could be used to form the composition of this invention, it has been found that a carrier comprising methacrylated sodium hyaluronate (MHy) or methacrylated hydroxyethylcellulose (MHEC) provide the most desirable properties.

A carrier comprising MHy is particularly preferred because it is highly biocompatible. Sodium hyaluronate is a safe, natural substance found in the joints of the human body, skin, and ocular fluids and plays a role in different physiological processes. For example, sodium hyaluronate is involved in biochemical mechanisms for healing wounds and relieving inflammation. The sodium hyaluronate may be functionalized with polymerizable groups, particularly methacrylate groups. The methacrylated sodium hyaluronate (MHy) or methacrylated hydroxyethylcellulose (MHEC) carriers are polymerized using polymerization initiators that generate free radicals upon being exposed to ultraviolet or visible light radiation. The free radicals initiate polymerization and cross-linking of the MHy and MHEC compounds to form a stable hydrogel matrix.

The methods used to synthesize the MHy and MHEC carrier materials are important as shown in the following Examples. MHy and MHEC carriers were synthesized in an aqueous solution of glycidyl methacrylate (GMA), triethylamine, tetrabutylammonium bromide, and sodium hyaluronate (Hy) or hydroxyethylcellulose (HEC) following the methods generally described in Leach, J.B., et al., Photocrosslinked Hyaluronic Acid Hydrogels: Natural, Biodegradable Tissue Engineering Scaffolds, Biotechnol Bioeng, 2003. 82(5): p. 578-89. Increasing the GM:Hy molar ratio increased the amount of derivatization.

In addition to the particulate bone grafting material and biodegradable, polymeric carrier, the composition contains a polymerization system that is activated by light to polymerize the carrier gel material. The polymerization system comprises a photopolymerization initiator that generates free radicals when irradiated with ultraviolet or visible light. Suitable polymerization initiators may include, but are not limited to, photosensitive dyes such as esosin, acridine, thiazine, xanthine, and phenazine dyes. For example, Eosin Y, acriblarine; thionine, rose bengal, and methylene blue dyes may be used. Other polymerization initiators may include benzophenone, benzoin and their derivatives or alpha-diketones and their derivatives. The photoinitiator also may be selected from the class of acylphosphine oxides such as, for example, 2,4,6-trimethylbenzoyl-diphenyl-phosphine oxide (TPO). Polymerization may be initiated by irradiating the.composition with energy in the ultraviolet or visible light spectrum having a wavelength generally in the range of 200 to 700 nm. Preferably, photopolymerization is initiated by irradiating the composition with blue, visible light preferably having a wavelength in the range of 400 to 600 nm. It is preferred that visible light be used, since there may be some adverse health effects with long term exposure to ultraviolet light. In addition, most dental offices contain standard light-curing units for irradiating other dental materials (for example, composites, adhesives, and sealants) with blue visible light. Such standard light-curing units can be used to irradiate the composition of this invention. The polymerization initiator absorbs the light and acts as a source of free radicals. The free radicals induce polymerization of the vinyl groups on the MHy or MHEC carrier. Polymerization accelerators, particularly tertiary amines, may be added to the composition to increase the rate of polymerization. For example, acrylate derivatives such as dimethylaminoethyl methacrylate, diethylaminoethyl methacrylate, and the like may be used. Also, aromatic tertiary amines such as, for example, N-methyl-diethanolamine, ethyl 4-(dimethylamino)benzoate (EDMAB), 2-[4-(dimethylamino)phenyl] ethanol, N, N-dimethyl-p-toluidine (DMPT), dihydroxyethyl-p-toluidine (DHEPT), bis(hydroxyethyl)-p-toluidine, triethanolamine (TEA), and the like may be used. In addition, compounds such as N-vinyl-2-pyrrolidone and N-vinyl caprolactam may be used as polymerization accelerators.

As discussed above, the composition of this invention is preferably in the form of putty having good viscosity and handling properties. This allows a clinician to mold and shape the composition to the desired structure at the bone repair site. The putty is moldable and adhesive at room temperature. It has good dimensional stability and maintains the particulate in suspension. The particulate is not allowed to migrate away from the bone repair site and this enhances new bone growth. Also, the putty is not immediately swelled by biological fluids and it does not dry out too quickly. In other embodiments, the composition may be in the form of a liquid, powder, semi-solid material, or the like.

The composition may be provided as two-part system with the particulate bone grafting material supplied in a first sterile package and the biodegradable, polymeric carrier loaded in a syringe which is supplied in a second sterile package. The first package is opened and the particulate material is poured into a sterile mixing container. Then, the clinician uses the syringe to dispense the polymeric carrier into the mixing container and onto the particles. The clinician mixes the carrier and particles thoroughly to form a homogenous putty-like mixture that is ready to be applied to the surgical site.

A sterile spatula or other suitable instrument can be used to apply the putty composition. Since the putty composition is substantially viscous and easy to handle, it can be molded into the required shape at the surgical site. Then, the composition may be photopolymerized by irradiating the composition with ultraviolet or visible light. As discussed above, it is preferred that visible light radiation be used to photopolymerize the composition. Standard blue light dental curing units may be used to irradiate the composition. The composition begins to gel and harden as the photopolymerization process is initiated. Within a relatively short period of time, the composition sets to form a stable cross-linked hydrogel.

The composition of this invention can be used in a variety of dental and orthopedic applications. For example, the composition can be used in sinus elevation defects, extraction sites, bone loss around implants and to support implant placement, extraction site ridge preservation, repair periodontal intrabony defects, pre-existing defects around implants, ridge augmentation, ridge onlay, repair furcation defects, to cover exposed implant surfaces or threads, or to repair an edentulous site to facilitate implant acceptance.

The composition of this invention, comprising a preferred particulate bone grafting material and polymeric carrier, has many advantageous features. Combining the preferred particulate material with the preferred carriers, methacrylated sodium hyaluronate (MHy) or methacrylated hydroxyethylcellulose (MHEC) produces a composition that is surprisingly effective. The composition has good handling properties and is capable of being cured in *vivo* by photopolymerization to form a stable hydrogel. The weight percentage of particles and carrier can be optimized to produce a composition that can be light-cured uniformly in a relatively short period of time. Thus, a clinician can easily mold the composition to a particularly desired shape and cure the structure *in vivo*. The resulting cured composition, while not being brittle, demonstrates higher compressive strength than light-curable DFDBA bone grafting materials.
The invention is further illustrated by the following Examples, but these Examples should not be construed as limiting the scope of the invention.

### EXAMPLES

The following **Examples 1-4A** describe different methods for synthesizing photopolymerizable sodium hyaluronate (Hy) and hydroxyethylcellulose (HEC).

### Example 1

Three different methods, as described below, were used to synthesize photopolymerizable sodium hyaluronate (Hy). The methods are referred to as Reactions I, II, and III.

### Reaction I. GMHy (methacrylated Hy) Synthesis

The method described in Leach, J.B., et al., Photocrosslinked Hyaluronic Acid Hydrogels: Natural, Biodegradable Tissue Engineering Scaffolds, Biotechnol Bioeng, 2003. 82(5): p. 578-89 was generally followed (hereinafter referred to as the "Leach Method") to prepare a GMHy carrier.

1% Hy was reacted with a 10-fold molar excess of glycidyl methacrylate and equal amounts of triethylamine and tetrabutyl ammonium bromide in water for 24 hours at room temperature. The reaction was continued at 60°C for 1 hour. Then, the solution was precipitated in acetone, dissolved in distilled (DI) water, precipitated a second time in acetone, and dissolved again in DI water to remove excess reactants. The GMHy solution was lyophilized and stored.

### Reaction II. Hy-MA (methacrylated Hy) Synthesis

The method described in Smeds, K.A., et al., Synthesis of a Novel Polysaccharide Hydrogel, Journal of Macromolecular Science 1999. A36(7&8): p. 981-989 was generally followed to prepare a Hy-MA carrier.

2% Hy was reacted with methacrylic anhydride at a pH of 8 for 24 hours at 5°C.

After the reaction, the solution was precipitated in ethanol, dissolved in DI water, precipitated a second time in ethanol, and dissolved again in DI water to remove excess reactants. The Hy-MA solution was lyophilized and stored.

### Reaction III. GARy (acrylated Hy) Synthesis

The Leach Method was generally followed to prepare GAHy macromer.

1% Hy was reacted with a 20-fold molar excess of glycidyl acrylate and equal amounts of triethylamine and tetrabutylammonium bromide in water for 24 hours at room temperature. The reaction was continued at 60°C for 1 hour. After the reaction, the solution was precipitated in acetone, dissolved in DI water precipitated a second time in acetone, and dissolved again in DI water to remove excess reactants. The GAHy solution was lyophilized and stored.

### Results

The synthesis of the GMHy carrier was successful. Gels made with approximately 8% GMHy were successfully photopolymerized. Hy-MA synthesis was successful, but hardened gels made with Hy-MA, with or without particulate mixed in, were all very brittle, even upon lowering the Hy-MA concentration. Additionally, both the reaction mixture and the derivatized material were opaque, indicating a solubility problem. GAHy synthesis was successful, though the material was very difficult to work with. It did not like to dissolve in water or buffer. Hardened gels made with GAHy, with or without particulate mixed in, were very brittle.

### Example 2

In the following Example, derivatization reactions were performed under various conditions to determine the effects of reactant concentration, composition, and pH on the derivatization of sodium hyaluronate (Hy) with glycidyl methacrylate. Hy is pH sensitive and the reaction conditions in the Leach Method are basic (pH 10.5-11), due to the addition of a phase transfer catalyst (tetrabutylammonium bromide) and a base (triethylamine). Because of concerns about Hy breaking down in these basic conditions, reactions were done with only glycidyl methacrylate and the phase transfer catalyst at a pH of 8.5, as well as with only glycidyl methacrylate at a pH of 7.2. Additionally, the effects of reactant concentration were investigated by conducting reactions with a glycidyl methacrylate molar ratio of 10, 15, and 20.

Table 1 shows the amounts of reactants used in each reaction, as well as the pH of the reaction. For Reactions IV and V, 1 g Hy was dissolved at 1% in either 12.5 mL 10 mM phosphate buffer, pH = 7.2 and 87.5 mL distilled (DI) water (to simulate Hyaluron's 8% Hy dissolved in DI water). For Reactions VI-XII, 1 g Hy was dissolved in 100 mL 10 mM phosphate buffer, pH = 7.2.

For each solution (Reactions I-XII), after the Hy was dissolved in water and/or buffer, and the remaining reagents were added and mixed well. All reactions were done at room temperature (RT) for 24 hours, followed by 1 hour incubation at 60°C. Derivatized Hy was then precipitated in acetone, dissolved in DI water, and precipitated a second time in acetone.

The components and conditions for the Reactions are shown in the following Table 1.

**Table 1 (Reaction Components and pH for Hy Derivatization Experiments)**

| Rxn. | Molar Ratio of GM | GM (mL) | TEA (mL) | TBAB (g) | pH |
|---|---|---|---|---|---|
| IV. | 15 | 5.4 | 5.4 | 5.4 | 9.6 - 11.1 |
| V. | 20 | 7.2 | 7.2 | 7.2 | 9.7 - 11.3 |
| VI. | 10 | 3.6 | 3.6 | 3.6 | 7.2 - 7.4 |
| VII. | 15 | 5.4 | | | 7.3 |
| VIII. | 20 | 7.2 | | | 7.3 |
| IX. | 10 | 3.6 | | 3.6 | 8.5 - 8.9 |
| X. | 15 | 5.4 | | 5.4 | 8.5 - 8.7 |
| XI. | 20 | 7.2 | | 7.2 | 8.3 - 8.7 |
| XII. | 10 | 3.6 | | | 7.2 - 7.4 |

All reactions resulted in photopolymerizable products. It was noted that the derivatized Hy prepared from Reactions VI-XII lacked stability when mixed with particulate bone grafting material. After the derivatized Hy prepared from Reactions VI-XII was mixed with the particulate material for one hour, it was no longer polymerizable or not polymerizable enough to make a hard product. This indicates a difference in reaction mechanisms during the derivatization.

There are at least two mechanisms by which glycidyl methacrylate may become conjugated to Hy as described in Li, Q., D.-a. Wang, and J.H. Elisseeff, Heterogeneous-Phase Reaction of Glycidyl Methacrylate and Chondroitin Sulfate: Mechanism of Ring-Opening-Transesterification Competition, Macromolecules, 2003. 36: p. 2556-2562. The first mechanism is transesterification, a rapid and reversible methacrylate-Hy conjugation. The second mechanism is a ring-opening mechanism where glycidyl methacrylate is irreversibly coupled to Hy. It is possible that in the lower pH reactions, the reversible mechanism is dominant, while with the higher pH reactions, the ring-opening mechanism dominates. This could be the source of the stability problems associated with derivatized Hy prepared from reactions at low pH.

### Example 3

In the following Example, derivatization reactions were performed under various conditions to determine the effects of increasing the reaction times (48 hours) and/or increasing the amount of glycidyl methacrylate added to the solution.

### Reaction XIII. GMHy (methacrylated Hy) Synthesis

The Leach Method was generally followed to prepare GMHy carrier.

1% Hy was reacted with a 10-fold molar excess of glycidyl methacrylate and equal amounts of triethylamine and tetrabutyl ammonium bromide in water for 48 hours at room temperature. The reaction was continued at 60°C for 1 hour. After the reaction, the solution was precipitated in acetone, dissolved in DI water precipitated a second time in acetone, and dissolved again in DI water to remove excess reactants. The GMHy solution was lyophilized and stored.

### Reaction XIV. GMHy (methacrylated Hy) Synthesis

The Leach Method was generally followed to prepare GMHy carrier

1% Hy was reacted with a 15-fold molar excess of glycidyl methacrylate and equal amounts of triethylamine and tetrabutylammonium bromide in water for 48 hours at room temperature. The reaction was continued at 60°C for 1 hour. After the reaction, the solution was precipitated in acetone, dissolved in DI water precipitated a second time in acetone, and dissolved again in DI water to remove excess reactants. The GMHy solution was lyophilized and stored.

### Reaction XV. GMHy (methacrylated Hy) Synthesis

The Leach Method was generally to prepare GMHy carrier.

1 % Hy was reacted with a 20-fold molar excess of glycidyl methacrylate and equal amounts of triethylamine and tetrabutylammonium bromide in water for 48 hours at room temperature. The reaction was continued at 60°C for 1 hour. After the reaction, the solution was precipitated in acetone, dissolved in DI water precipitated a second time in acetone, and dissolved again in DI water to remove excess reactants. The GMHy solution was lyophilized and stored.

### Reaction XVI. GMHy (methacrylated Hy) Synthesis

The Leach Method was generally to prepare GMHy carrier.

1% Hy was reacted with a 30-fold molar excess of glycidyl methacrylate and equal amounts of triethylamine and tetrabutylammonium bromide in water for 24 hours at room temperature. The reaction was continued at 60°C for 1 hour. After the reaction, the solution was precipitated in acetone, dissolved in DI water precipitated a second time in acetone, and dissolved again in DI water to remove excess reactants. The GMHy solution was lyophilized and stored.

### Reaction XVII. GMHy (methacrylated Hy) Synthesis

The Leach Method was generally followed to prepare GMHy carrier.

1% Hy was reacted with a 30-fold molar excess of glycidyl methacrylate and equal amounts of triethylamine and tetrabutylammonium bromide in water for 48 hours at room temperature. The reaction was continued at 60°C for 1 hour. After the reaction, the solution was precipitated in acetone, dissolved in DI water precipitated a second time in acetone, and dissolved again in DI water to remove excess reactants. The GMHy solution was lyophilized and stored.

### Example 4

In the following Example, photopolymerizable hydroxyethylcellulose (HEC) was prepared in accordance with the Leach Method.

### Reaction XVIII. GMHy (methacrylated Hy) Synthesis

1% HEC was reacted with a 10-fold molar excess of glycidyl methacrylate and equal amounts of triethylamine and tetrabutyl ammonium bromide in water for 24 hours at room temperature. The reaction was continued at 60°C for 1 hour. After the reaction, the solution was precipitated in acetone, dissolved in DI water precipitated a second time in acetone, and dissolved again in DI water to remove excess reactants. The GMHEC solution was lyophilized and stored.
In this Example 4, HEC was derivatized with methacrylate groups successfully. Photopolymerization with Eosin Y initiating systems was successful. However, the resulting GMHEC hydrogels were both harder and more brittle than GMHy hydrogels.

### Example 4A

In an attempt to make the GMHEC hydrogels, as produced according to Example 4, less brittle, a reaction was carried out using a lower concentration of glycidyl methacrylate. The reaction was carried out under the following conditions.

### Reaction XIX. GMHy (methacrylated Hy) Synthesis

1% Hy was reacted with one-half the amount of glycidyl methacrylate, triethylamine and tetrabutyl ammonium bromide used in above Example 4 (Reaction XVIII) in water for 24 hours at room temperature. The reaction was continued at 60°C for 1 hour. After the reaction, the solution was precipitated in acetone, dissolved in DI water precipitated a second time in acetone, and dissolved again in DI water to remove excess reactants. The GMHEC solution was lyophilized and stored.

In this Example 4A, the resulting material, when polymerized, was less hard than the original GMHEC, but not less brittle.

### Review of Reactions I-XIX

Based on the foregoing, Reactions I and IV are the preferred reactions for making a biocompatible and biodegradable polymeric carrier that can be used in the composition of this invention. Reactions V and XVIII also can be used to produce a polymeric carrier having good properties. All other Reactions, as described above, are less preferred methods for synthesizing the carrier.

The following Example 5 describes polymerizable compositions with different polymerization initiator systems.

### Example 5

In this Example, different compositions comprising GMHy and polymerization initiators were prepared. The compositions did not contain any bone grafting material. The compositions are described in the following Tables 2-4. The compositions were polymerized using a Dentsply Spectrum 800 dental lamp (Dentsply International) and the results are reported below.

Formulations were prepared containing differing amounts of the photopolymerization initiator dye, Eosin Y (EY); triethanolamine (TEA); and N-vinyl-2-pyrrolidone (NVP) as described in the following Table 2.

**Table 2 (Photopolymerizable Formulations Containing Eosin Y Initiator)**

| Formulation: | EY (mM): | TEA (mM): | NVP (%): |
|---|---|---|---|
| A | 0.059 | 820 | 0.82 |
| B | 5 | 100 | 0.5 |
| C | 0.5 | 100 | 0.5 |
| D | 0.1 | 100 | 0.5 |
| E | 0.05 | 100 | 0.5 |
| F | 0.025 | 100 | 0.5 |
| G | 0.2 | 200 | 1.0 |
| H | 0.1 | 200 | 1.0 |
| I | 0.05 | 200 | 1.0 |
| J | 0.05 | 50 | 0.25 |
| K | 0.025 | 50 | 0.25 |
| L | 0.013 | 50 | 0.25 |

Secondly, formulations containing camphorquinone (CQ) were prepared. However, CQ is not soluble in water. Dissolution of CQ in ethanol is possible, but polymerization doesn't occur. (Hy is not soluble in ethanol.) Even a very dilute CQ solution in water did not work. Because the photopolymerizable gel is made of water, a hydrophilic initiator is necessary.

Thirdly, formulations containing the photopolymerization initiator dye, Rose Bengal (RB) were prepared as described in the following Table 3.

**Table 3 (Photopolymerizable Formulations Containing Rose Bengal Initiator)**

| Formulation: | RB (mM): | TEA (mM): | NVP (%): |
|---|---|---|---|
| M | 0.5 | 100 | 0.5 |
| N | 0.05 | 100 | 0.5 |

Lastly, formulations containing N-vinyl-2-pyrrolidone (NVP) or N-vinyl caprolactam (NVC) were prepared as described in Table 4.

**Table 4 (Photopolymerizable Formulations Containing NVP or NVC Initiators)**

| Formulation: | EY (mM): | TEA (mM): | NVP (%): | NVC (%): |
|---|---|---|---|---|
| O | 0.025 | 100 | 0 | 0.5 |
| P | 0.025 | 100 | 0 | 1.0 |
| Q | 0.025 | 200 | 0 | 0.5 |
| R | 0.025 | 200 | 0 | 1.0 |
| S | 0.025 | 100 | 0.5 | 0 |
| T | 0.025 | 100 | 1.0 | 0 |
| U | 0.025 | 200 | 0.5 | 0 |
| V | 0.025 | 200 | 1.0 | 0 |

Because NVC is less soluble in water than NVP, the initial initiator solution that was made, before mixing with the macromer gel, was more dilute. For formulations containing NVC, 100 µL of initiator solution per g of macromer gel was used, while for formulations containing NVP, 50 µL of initiator solution per g of macromer gel was used.

### Results

Based on cure depth data, qualitative physical properties of cured gels, and FDA acceptance history of components, an initiating system comprising of 0.025 mM Eosin Y (EY), 100 mM triethanolamine (TEA), and 0.5% n-vinyl caprolactam (NVC) is the most preferred.

The following Example 6 describes different compositions containing GMHy hydrogel and particulate bone graft materials.

### Example 6

In this Example, different compositions comprising GMHy hydrogel carrier and particulate bone graft materials, DFDBA or PepGen P-15™ were prepared. The concentration of GMHY (in buffer) was varied as well as the particulate concentrations. These compositions are described in the following Table 5. The composition was polymerized using a Dentsply Spectrum 800 dental lamp (Denstply International) and the results are reported below.

**Table 5 (Photopolymerizable Compositions Containing Particulate Bone Graft Material and Carrier)**

| Formulation: | GMHy % | PepGen P-15™ wt % | DFDBA wt% | Particulate Vol % |
|---|---|---|---|---|
| 1 | 8 | 37 | - | 15 |
| 2 | 6 | 37 | - | 15 |
| 3 | 4 | 37 | - | 15 |
| 4 | 8 | 55 | - | 27 |
| 5 | 6 | 55 | - | 27 |
| 6 | 4 | 55 | - | 27 |
| 7 | 8 | 40 | - | 17 |
| 8 | 8 | 45 | - | 20 |
| 9 | 8 | 50 | - | 24 |
| 10 | 8 | - | 18 | 17 |
| 11 | | | 21 | 20 |
| 12 | | | 25 | 24 |
| 13 | 8 | - | 29 | 27 |

Formulations containing 8 wt.% GMHy are the most preferred. Gels containing 4-6 wt.% GMHY are sticky before curing and not as solid after curing. Among the 8% formulations, those with lower particulate concentrations are not as hard as those formulations with higher concentrations, but the depth to which they cure during light exposure is higher. All compositions having particulate concentrations in the range of 37-55 wt.% are effective, but they have different properties both before and after light curing.

18 wt.% DFDBA formulations have the same volume % particulate as 40 wt.% PepGen P-15™ formulations. Table 5 shows vol.% and wt.% values for each particulate formulation. Formulations made with DFDBA have similar handling properties before curing, compared to PepGen P-15™ formulations. After curing, the DFDBA formulations are softer and don't cure as far down as the PepGen P-15™ formulations. For this reason, Formulations with PepGen P-15™ are preferable.

The preferred GMHy/Particulate compositions are identified as Formulation Nos.: 4, 7, 8, and 9 in above Table 5.

### Mechanical Testing of Compositions Containing Various Particulate Bone Grafting Materials and Carriers

The compressive modulus of compositions described above were measured with a texture analyzer (n= 4-5) and the results are reported in the following bar graphs.

As discussed above, OG/N-300 is an anorganic bone matrix (ABM) particulate material and is the same particulate material used in the PepGen P-15™ product. Because the same particulate is used in the OG/N-300 and PepGen P-15™ materials, the products behave the same in terms of physical and mechanical properties. In the following mechanical property tests, OG/N-300 material was used as the test sample.

### Test 1 (Compressive Modulus Comparison of Materials Made with DFDBA vs. Materials Made With OG/N-300 at Various Particulate Concentrations):

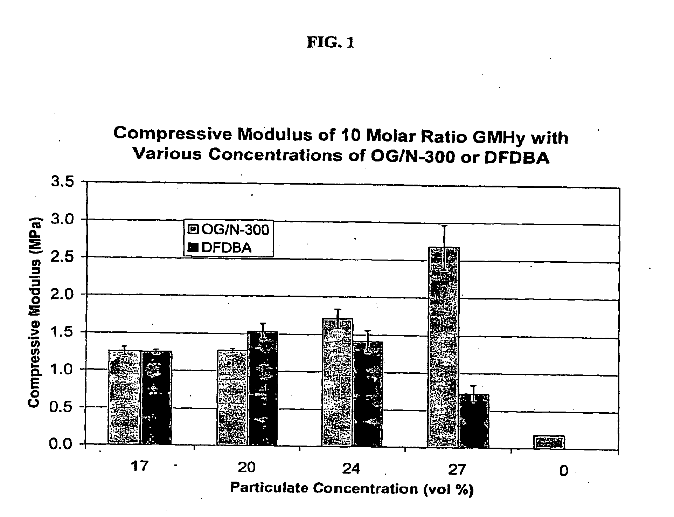

In this Test 1, the GMHy carrier was synthesized according to the method described in Example 1 (Reaction I); the polymerization system was Formulation O (Table 4, Example 5); and Formulations: 4, 7-13 (Example 6); and Formulations 4 and 7-13 (Example 6) were tested. The results are shown in the bar graph of FIG. 1.

### Test 2 (Compressive Modulus Comparison of Materials Made with DFDBA vs. Materials Made with ABM at Various Cure Times):

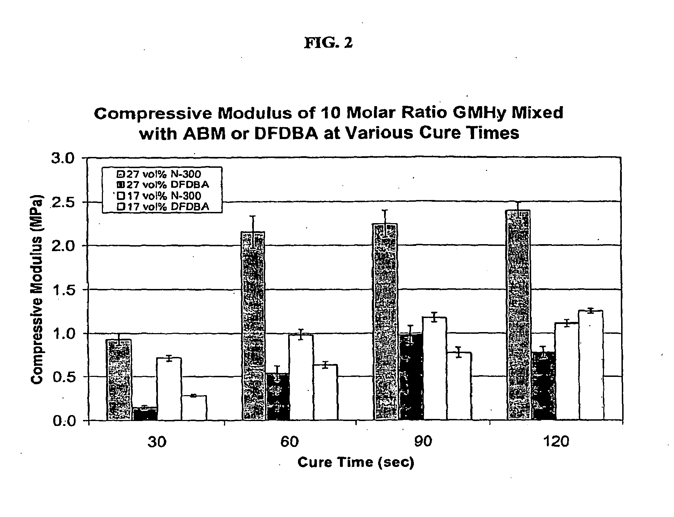

In this Test 2, the GMHy carrier was synthesized according to the method described in Example 1 (Reaction I); the polymerization system was Formulation O (Table 4, Example 5); and Formulations 4 and 7-13 (Example 6) were tested. The results are shown in the bar graph of FIG. 2.

### Test 3 (Compressive Modulus Comparison of Two Materials Made with OG/N-300 at Various Particulate Concentrations and Cure Times):

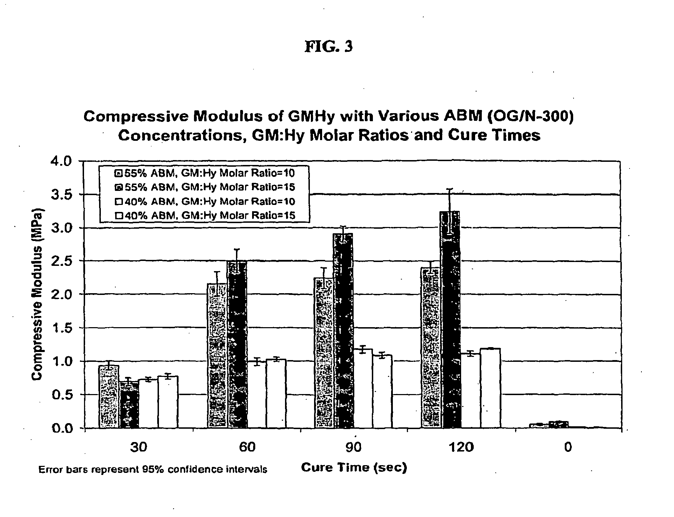

In this Test 3, the GMHy carrier was synthesized according to the method described in Example 1 (Reaction I); the polymerization system was Formulation O (Table 4, Example 5); and Formulations 4 and 7 (Example 6) were tested. The results are shown in the bar graph of FIG. 3.

### Test 4 (Compressive Modulus Comparison of Two Materials Made with Various Concentrations of GMHy Carrier and OG/N-300):

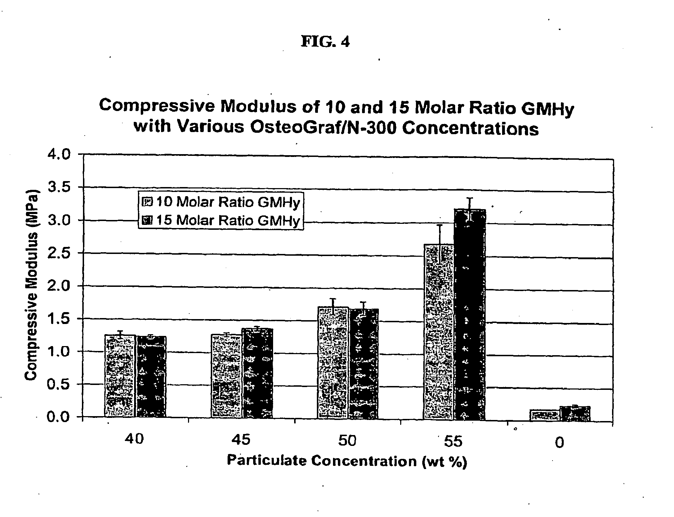

The GMHy carrier was synthesized according to the methods described in Examples 1 and 2 (Reactions I and IV); the polymerization system was Formulation O (Table 4, Example 5); and Formulations 4 and 7-9 (Example 6) were tested. The results are shown in the bar graph of FIG.4.

### RABBIT BONE IMPLANTATION STUDIES

### Study 1

Bilateral, unicortical tibial defects, 5mm in diameter, were created in four New Zealand (NZ) rabbits. Sites were grafted with light-cured putty material composed of anorganic bone matrix coated with P-15 cell binding peptide (ABM/P-15) in either GMHEC or GMHy. Grafts were cured in situ with visible light. After six weeks, histology and radiography were performed.

The GMHy carrier was synthesized according to the methods described in Examples 2 and 4 (Reactions XII and XVIII); the polymerization system was Formulation O (Table 4, Example 5); and Formulation 4 was tested.

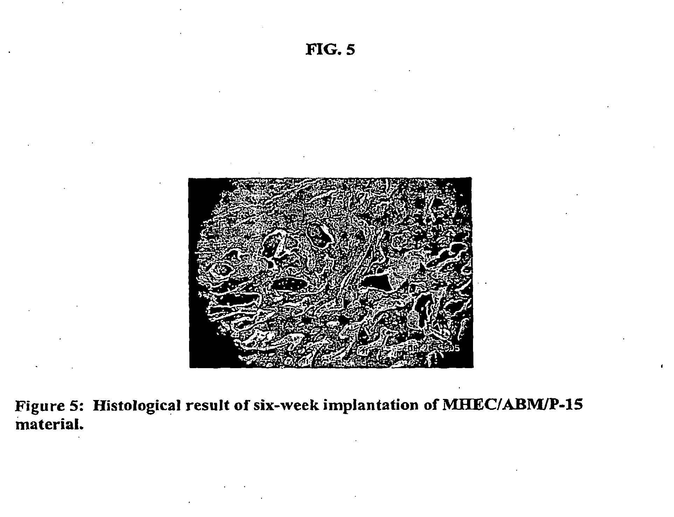

Histological and radiographic results showed moderate to abundant bone formation within both graft materials, in all defects. Mild to moderate inflammation was observed. Figure 5 shows a representative histological result of bone formation six weeks after implantation and in situ curing of light-curable hydrogel/ABM/P-15 graft material.

### Study 2

In a second study, bone formation at four weeks with uncured and in situ cured GMHy were compared in bilateral 2.0 mm diameter defects in the femurs of six NZ White Rabbits.

The GMHY carrier was synthesized according to the method described in Example 2 (Reaction 1); the polymerization system was Formulation O (Table 4, Example 5); and Formulation 4 (Example 6) was tested.
Both cured and uncured test materials were well tolerated in the bone and adjacent muscle tissue. Uncured material resulted in slightly better defect repair and osteo-integration than the cured material. Both materials were considered to be nonirritants to the muscle overlying the bone.

## Claims

1. A composition for promoting growth of new bone material, comprising:
porous, resorbable particulate derived from anorganic bone material;
resorbable, biocompatible carrier gel material comprising photopolymerizable polysaccharide having polymerizable groups, said material forming a stable hydrogel matrix upon polymerization, said particulate being dispersed within said hydrogel; and
a polymerization system that is activated by light to polymerize the carrier gel material.

2. The composition of claim 1, wherein said particulate is bovine-derived and has an average particle size in the range of 250 µm to 1000 µm, preferably in the range of 250 µm to 420 µm.

3. The composition of claim 1, wherein said particulate is present in an amount in the range of 30% to 75% by weight based on weight of said composition.

4. The composition of claim 3, wherein said particulate has a bulk density of 1.1 to 1.3 g/cc and the composition comprises 40% to 60% particulate.

5. The composition of claim 1, wherein said carrier gel comprises methacrylated sodium hyaluronate, methacrylated hydroxyethylcellulose, or a mixture of methacrylated sodium hyaluronate and methacrylated hydroxyethylcellulose.

6. The composition of claim 1, wherein said carrier gel comprises 2 to 10% by weight photopolymerizable polysaccharide.

7. The composition of claim 1, wherein the polymerization system comprises a photopolymerization initiator selected from the group consisting of Eosin Y, acriblarine, thionine, rose Bengal, and methylene blue dyes, and mixtures thereof.

8. The composition of claim 1, wherein the polymerization system is activated by blue, visible light having a wavelength in the range of 400 to 600 nm.

9. The composition of claim 1, wherein the polymerization system comprises a photopolymerization accelerator selected from the group consisting of N-methyl-diethanolamine, ethyl 4-(dimethylamino)benzoate (EDMAB), 2-[4-(dimethylamino)phenyl] ethanol, N,N-dimethyl-p-toluidine (DMPT), dihydroxyethyl-p-toluidine (DHEPT), bis(hydroxyethyl)-p-toluidine, triethanolamine (TEA), and mixtures thereof.

10. The composition of claim 1, wherein the polymerization system comprises a photopolymerization accelerator selected from the group consisting of N-vinyl-2-pyrrolidone, N-vinyl caprolactam, and mixtures thereof.

11. The composition of claim 1, wherein the polymerization system comprises a blend of Eosin Y, triethanolamine, and N-vinyl caprolactam.

12. The composition of claim 1, wherein said porous, resorbable particulate is bound to P-15 polypeptide material.

13. Use of a porous, resorbable particulate derived from anorganic bone material; resorbable, biocompatible carrier gel material comprising photopolymerizable polysaccharide having polymerizable groups, said material forming a stable hydrogel matrix upon polymerization, said particulate being dispersed within said hydrogel; and a polymerization system that is activated by light to polymerize the carrier gel material for the preparation of a bone growth inducing composition for treating defective bone tissue.

14. The use of claim 13, wherein the composition has putty-like consistency.

## Patentansprüche

1. Zusammensetzung zur Förderung des Wachstums von neuem Knochenmaterial, umfassend:
poröse, resorbierbare Teilchen, die von anorganischem Knochenmaterial abgeleitet sind;
ein resorbierbares, biokompatibles Träger-Gelmaterial, umfassend ein photopolymerisierbares Polysacharid mit polymerisierbaren Gruppen,
wobei das Material durch Polymerisation eine stabile Hydrogelmatrix bildet, wobei die Teilchen in dem Hydrogel dispergiert sind; und
ein Polymerisationssystem, das durch Licht aktiviert wird, um das Trägergelmaterial zu polymerisieren.

2. Die Zusammensetzung nach Anspruch 1, wobei die Teilchen vom Rind abgeleitet sind und eine mittlere Teilchengröße im Bereich von 250 µm bis 1000 µm, vorzugsweise im Bereich von von 250 µm bis 420 µm, aufweisen.

3. Die Zusammensetzung nach Anspruch 1, wobei die Teilchen in einer Menge im Bereich von 30 Gew.% bis 75 Gew.%, bezogen auf das Gewicht der Zusammensetzung, vorliegen.

4. Die Zusammensetzung nach Anspruch 3, wobei die Teilchen eine Schüttdichte von 1,1 bis 1,3 g/cc aufweisen und die Zusammensetzung 40% bis 60% Teilchen umfasst.

5. Die Zusammensetzung nach Anspruch 1, wobei das Trägergel methacryliertes Natriumhyaluronat, methacrylierte Hydroxyethylcellulose oder ein Gemisch aus methacryliertem Natriumhyaluronat und methacrylierter Hydroxyethylcellulose umfasst.

6. Die Zusammensetzung nach Anspruch 1, wobei das Trägergel 2 bis 10 Gewichts-% photopolymerisierbares Polysacharid umfasst.

7. Die Zusammensetzung nach Anspruch 1, wobei das Polymerisationssystem einen Photopolymerisationsinitiator umfasst, der ausgewählt ist, aus der Gruppe bestehend aus Eosin Y-, Acriblarin-, Thionin-, Bengal-Rosa- und Methylenblau-Farbstoffen sowie Gemischen davon.

8. Die Zusammensetzung nach Anspruch 1, wobei das Polymerisationssystem durch blaues, sichtbares Licht einer Wellenlänge im Bereich 400 bis 600 nm aktiviert wird.

9. Die Zusammensetzung nach Anspruch 1, wobei das Polymerisationssystem einen Photopolymerisationsbeschleuniger umfasst, der ausgewählt wird aus der Gruppe, bestehend aus N-Methyldiethanolamin, Ethyl 4-(dimethylamino)benzoat (EDMAB), 2-[4-(Dimethylamino)phenyl]ethanol, N,N-Dimethyl-p-toluidin (DMPT), Dihydroxyethyl-p-toluidin (DHEPT), Bis(hydroxyethyl)-p-toluidin, Triethanolamin (TEA) sowie Gemischen davon.

10. Die Zusammensetzung nach Anspruch 1, wobei das Polymersiationssystem einen Photopolymerisations-beschleuniger umfasst, der ausgewählt wird aus der Gruppe bestehend aus N-Vinyl-2-pyrrolidon, N-vinylcaprolactam sowie Gemischen davon.

11. Die Zusammensetzung nach Anspruch 1, wobei das Polymerisationssystem ein Gemisch aus Eosin Y, Triethanolamin und N-vinyl-Caprolactam umfasst.

12. Die Zusammensetzung nach Anspruch 1, wobei die porösen, resorbierbaren Teilchen an P-15-Polypeptidmaterial gebunden sind.

13. Verwendung von porösen resorbierbaren Teilchen, die abgeleitet sind von anorganischem Knochenmaterial; einem resorbierbaren biokompatiblen Träger-Gelmaterial, umfassend photopolymerisierbares Polysacharid mit polymerisierbaren Gruppen, wobei das Material eine stabile Hydrogelmatrix nach Polymerisation bildet, wobei die Teilchen in dem Hydrogel dispergiert sind; sowie eines Polymerisationssystems, das durch Licht aktiviert wird, um das Trägergelmaterial zu polymerisieren, zur Herstellung einer knochenwachstumsfördernden Zusammensetzung zur Behandlung von schadhaftem Knochengewebe.

14. Die Verwendung nach Anspruch 13, wobei die Zusammensetzung eine knetgummiartige Konsistenz aufweist.

## Revendications

1. Composition pour favoriser la croissance de néo-substance osseuse comprenant :
un matériau particulaire poreux, résorbable, dérivé d'un matériau osseux inorganique ;
un matériau gel support, biocompatible, comprenant
un polysaccharide photopolymérisable ayant des groupes polymérisables, ledit matériau formant une matrice d'hydrogel stable après polymérisation, ledit matériau particulaire étant dispersé à l'intérieur dudit hydrogel ; et
un système de polymérisation activé par la lumière pour polymériser le matériau gel support.

2. Composition selon la revendication 1, dans laquelle ledit matériau particulaire est dérivé de bovins et a une taille moyenne de particules se situant entre 250 µm et 1000 µm, de préférence se situant entre 250 µm et 420 µm.

3. Composition selon la revendication 1, dans laquelle ledit matériau particulaire est présent en une quantité se situant entre 30 % et 75 % en poids de ladite composition.

4. Composition selon la revendication 3, dans laquelle ledit matériau particulaire a une densité apparente de 1,1 à 1,3 g/cc et la composition comprend de 40 % à 60 % de matériau particulaire.

5. Composition selon la revendication 1, dans laquelle ledit gel support comprend du hyaluronate de sodium méthacrylé, de l'hydroxyéthylcellulose méthacrylée ou un mélange de hyaluronate de sodium méthacrylé et d'hydroxyéthylcellulose méthacrylée.

6. Composition selon la revendication 1, dans laquelle ledit gel support comprend de 2 à 10 % en poids de polysaccharide photopolymérisable.

7. Composition selon la revendication 1, dans laquelle le système de polymérisation comprend un initiateur de photopolymérisation choisi dans le groupe constitué par les colorants éosine Y, acriblarine, thionine, rose Bengale, et bleu de méthylène, et des mélanges de ceux-ci.

8. Composition selon la revendication 1, dans laquelle le système de polymérisation est activé par une lumière bleu visible, ayant une longueur d'onde dans la plage allant de 400 à 600 nm.

9. Composition selon la revendication 1, dans laquelle le système de polymérisation comprend un accélérateur de photopolymérisation choisi dans le groupe constitué par la N-méthyl-diéthanolamine, le 4-(diméthylamino)benzoate d'éthyle (EDMAB), le 2-[4-(diméthylamino)phényl] éthanol, la N,N-diméthyl-p-toluidine (DMPT), la dihydroxyéthyl-p-toluidine (DHEPT), la bis(hydroxyéthyl)-p-toluidine, la triéthanolamine (TEA), et des mélanges de ceux-ci.

10. Composition selon la revendication 1, dans laquelle le système de polymérisation comprend un accélérateur de photopolymérisation choisi dans le groupe constitué par la N-vinyl-2-pyrrolidone, le N-vinyl caprolactam, et des mélanges de ceux-ci.

11. Composition selon la revendication 1, dans laquelle le système de polymérisation comprend un mélange d'éosine Y, de triéthanolamine et de N-vinyl caprolactam.

12. Composition selon la revendication 1, dans laquelle ledit matériau particulaire, poreux, résorbable est lié à un matériau à base de polypeptide P-15.

13. Utilisation d'un matériau particulaire, poreux, résorbable dérivé d'un matériau osseux inorganique ; d'un matériau gel support, biocompatible, comprenant un polysaccharide photopolymérisable ayant des groupes polymérisables, ledit matériau formant une matrice d'hydrogel stable après polymérisation, ledit matériau particulaire étant dispersé à l'intérieur dudit hydrogel ; et un système de polymérisation activé par la lumière pour polymériser le matériau gel support pour la préparation d'une composition induisant une croissance osseuse afin de traiter des défauts du tissu osseux.

14. Utilisation selon la revendication 13, dans laquelle la composition a une consistance semblable à celle du mastic.
